# EUROPEAN PATENT SPECIFICATION

(11) **EP 3 096 718 B1**
(45) Date of publication and mention of the grant of the patent: **26.08.2020**
(21) Application number: 15700610.7
(22) Date of filing: 22.01.2015
(51) Int. Cl.: A61F 5/441, A61L 9/014, A61L 15/00

(54) **OSTOMY BAG FILTER WITH ADHESIVE WEBBING**
OSTOMIEBEUTELFILTER MIT HAFTGEWEBE
FILTRE DE SAC DE STOMIE POURVU D'UNE SANGLE ADHÉSIVE

(30) Priority: 22.01.2014 GB 201401031
(43) Date of publication of application: 30.11.2016
(73) Proprietor: Welland Medical Limited, Crawley, West Sussex RH10 9TU (GB)
(72) Inventor: TROY, Padraig Gerard, Crawley West Sussex RH10 9TU (GB)
(74) Representative: Lock, Graham James
(86) International application number: PCT/EP2015/051276
(87) International publication number: WO 2015/110544

(56) References cited:
- EP-A1- 2 151 219
- EP-A2- 0 358 316
- EP-A2- 0 443 728
- EP-A2- 1 757 252
- GB-A- 2 215 605

## Description

The present invention relates to a filter and in particular to an ostomy bag comprising a filter of the invention.

### Background

Ostomy bags for receiving bodily waste from colostomy or ileostomy patients are well known. One problem with existing bags is that of odour release. When waste gases accumulate within such bags, the gases may be released through various mechanisms, and this gaseous release may cause an unpleasant odour.

In order to prevent odour release, some known ostomy bags include a filter to adsorb odours and through which waste gases may be released.

However, there are certain disadvantages with the products that have been developed to date. In such ostomy bags including a filter, the filter comprises an activated carbon layer, usually in the form of an impregnated foam, and a white PTFE layer. The filter is affixed to the bag by welding the PTFE layer of the filter directly to the inner layer of the ostomy bag. This direct welding of the filter to the inner layer of the ostomy bag can damage the material of the ostomy bag inner layer where it is welded, causing a point of weakness, which can lead to rupture of the bag layer and/or a leakage path through which odour can permeate. In addition, if adhesive is used, the layer of adhesive can act as a barrier to the gas, so that the gas cannot permeate the adhesive but must pass around it reducing the effectiveness of the filter. Similar problems can occur using other methods of adhering the filter to the ostomy bag inner layer.

For example, GB 2215605 discloses a filter for an ostomy bag that includes a number of components laminated together. The layers are assembled in the following order: (a) a layer of hot-melt adhesive, (b) a layer of microfine non-woven material, (c) a matrix layer of hot-melt adhesive, (d) a filter member, (e) a matrix layer of hot-melt adhesive, and (f) a layer of non-woven fabric. The filter is affixed to a wall of the bag using the layer (a) of hot-melt adhesive. Other filters for ostomy bags, such as that disclosed in WO 2009/146076, include an adhesive zone containing an exit window portion on the side of the filter assembly to be affixed to the bag, as well as a weld area surrounding the adhesive area. EP 358 316 A2 discloses a filter for an ostomy bag comprising a backing layer, a layer of odour-adsorbing material and a layer of adhesive webbing. EP 0 443 728 A2 discloses a filter for an ostomy bag comprising a backing layer, a layer of odour-adsorbing material and a layer of adhesive. EP 2 151 219 A1 discloses a filter for an ostomy bag comprising a backing layer, a layer of odour-adsorbing material and a breathable waterproof layer. EP 1 757 252 A2 discloses a filter for an ostomy bag comprising a layer of odour-adsorbing material and a hydrophobic support layer.

The present invention sets out to provide filters for ostomy bags which act to adsorb odours and thus prevent odour release during use, as well as improved ostomy bags incorporating such filters.

### Summary of the Invention

The invention is defined by claim 1. Preferred embodiments are defined by the dependent claims. In accordance with the present invention, there is provided a filter for an ostomy bag comprising a backing layer, a layer of odour-adsorbing material, a hydrophobic membrane layer, and a layer of adhesive webbing, comprising distinct fine lines of adhesive.

The use of a layer of adhesive webbing in the filter of the invention allows the filter to be adhesively bonded to the ostomy bag in a secure manner, and when in place allows gas to permeate through the holes in the webbing. In addition, the use of a fine webbing of adhesive minimises damage to the material of the ostomy bag, thus preventing or minimising leakage.

The following definitions shall apply throughout the specification and the appended claims.

Within the context of the present specification, the term "comprises" is taken to mean "includes" or "contains", i.e. other integers or features may be present, whereas the term "consists of" is taken to mean "consists exclusively of".

Within the context of the present specification, the term "about" is interpreted to mean optionally ±20%, preferably optionally ±10%, more preferably optionally ±5%.

Preferably, the backing layer comprises a fabric layer, a crushed foam layer, a film layer, a non-woven fabric layer, or any combination thereof. In one preferred embodiment, the backing layer comprises a layer of thermally bonded non-woven fabric.

Preferably, the backing layer has a thickness of at least 5 µm, at least 10 µm, or at least 15 µm. Preferably, the backing layer has a thickness of up to 100 µm, up to 150 µm, or up to 200 µm. In one embodiment of the invention, the backing layer has a thickness of from 5 µm to 200 µm. In another embodiment of the invention, the backing layer has a thickness of from 15 µm to 100 µm.

In one embodiment of the invention, the backing layer is permeable. In another embodiment of the invention, the backing layer is semi-permeable. In a further embodiment of the invention, the backing layer is impermeable.

Preferably, the layer of odour-adsorbing material comprises activated carbon. Preferably, the layer of odour-adsorbing material comprises an activated carbon-impregnated non-woven fabric, an activated carbon-impregnated woven fabric, an activated carbon-impregnated cloth, an activated carbon-impregnated felt, an activated carbon-impregnated knitted fabric, an activated carbon cloth, an activated carbon felt, and/or an activated carbon fabric. In one preferred embodiment of the invention, the layer of odour-adsorbing material comprises an activated carbon-impregnated foam, for example a polyurethane foam. In one particularly preferred embodiment of the invention, the layer of odour-adsorbing material comprises a polyurethane foam sprayed with chemically activated carbon. In another embodiment of the invention, the layer of odour-adsorbing material comprises a treated activated carbon, or a blend of treated activated carbon and activated carbon. Such a blend of treated activated carbon and activated carbon provides for both physisorption and chemisorption of odours.

Preferably, the layer of odour-adsorbing material has a thickness of at least 0.1 mm, at least 0.3 mm, or at least 0.5 mm. Preferably, the layer of odour-adsorbing material has a thickness of up to 3 mm, up to 4 mm, or up to 5 mm. In one embodiment of the invention, the layer of odour-adsorbing material has a thickness of from about 0.1 mm to about 5 mm. In another embodiment of the invention, the layer of odour-adsorbing material has a thickness of from about 0.5 mm to about 3 mm.

Preferably, the hydrophobic membrane layer comprises a material that is both hydrophobic and oleophobic. Preferably, the hydrophobic membrane layer comprises a material selected from polytetrafluoroethylene (PTFE), expanded PTFE (ePTFE), polyvinylidene difluoride (PVdF), polysulfone (PSu), polyethersulfone (PES), acrylic, nylon, polyoxymethylene (POM) (also known as acetal), a polyolefin, cellulose, and a combination thereof. In one preferred embodiment, the hydrophobic membrane layer comprises polytetrafluoroethylene (PTFE) or expanded PTFE (ePTFE). PTFE or ePTFE is preferred because these materials arehydrophobic and also have fairly high heat resistance, which are useful properties, particularly if used in conjunction with a hot-melt adhesive.

Preferably, the hydrophobic membrane layer has a thickness of at least 25 µm. Preferably, the hydrophobic membrane layer has a thickness of up to 200 µm. In one embodiment of the invention, the hydrophobic membrane layer has a thickness of from 25 µm to 200 µm.

Preferably, the hydrophobic membrane layer comprises a porous material. In this embodiment, the average pore size is preferably from at least about 0.05 µm up to about 5.0 µm. The hydrophobic membrane layer may additionally comprise a support material to assist in handling and sealing. Preferred support materials include non-woven polyester. In one preferred embodiment of the invention, the hydrophobic membrane layer comprises ePTFE on a non-woven polyester support.

A layer of adhesive webbing is provided on the surface of the hydrophobic membrane layer. The adhesive webbing layer comprises a plurality of distinct fine lines of adhesive with gaps or holes in the layer. For example, the lines of adhesive may be arranged in a grid-like pattern. The purpose of the adhesive is to bond the filter to a layer of the ostomy bag with which it is to be used. The use of an adhesive webbing allows gas to permeate through the gaps or holes in the webbing layer improving the effectiveness of the filter.

Preferably, the adhesive webbing layer comprises a hot melt adhesive. A hot melt adhesive is preferred because no drying or curing step is necessary, the use of volatile organic compounds (VOCs) is avoided, and there is no shrinkage during drying.

Preferably, the adhesive webbing layer comprises a vinyl acetate adhesive. The melting point of vinyl acetate adhesives is generally close to the melting point of materials commonly used in the manufacture of ostomy bags. This matching of the melting points enables the optimum welding of the filter to an ostomy bag.

The layers of the filter may be affixed to one another by any known method. For example, the layers of the filter may be affixed to one another using adhesive layers. In one embodiment of the invention, a layer of adhesive is provided between the backing layer and the layer of odour-adsorbing material. In another embodiment of the invention, a layer of adhesive is provided between the layer of odour-adsorbing material and the hydrophobic membrane layer. In one preferred embodiment of the invention, a first layer of adhesive is provided between the backing layer and the layer of odour-adsorbing material, and a second layer of adhesive is provided between the layer of odour-adsorbing material and the hydrophobic membrane layer. Preferably, the layers of adhesive comprise a polyamide adhesive. More preferably, the layers of adhesive comprise a polyamide adhesive web.

The filter of the invention may be fabricated to any desired size and shape. In one embodiment of the invention, the filter is disc-shaped. In this embodiment, the disc-shaped filter preferably has a diameter of from about 15 mm to about 40 mm, more preferably from about 20 mm to about 35 mm. In one preferred embodiment of the invention, the disc-shaped filter has a diameter of about 24.4 mm. The filter according to the invention preferably has an overall thickness of from about 2 mm to about 4 mm. In one preferred embodiment of the invention, the filter has a thickness of about 2.7 mm.

The filter of the invention may be used in conjunction with any type of ostomy bag. For example, the filter may be used in conjunction with a conventional disposable ostomy bag or with a flushable ostomy bag. Certain existing ostomy bags with which a filter of the invention may be used are described in WO 2010/122314, EP 2289471, EP 2476398, WO 2012/095637, and WO 2012/095638.

Accordingly, the present invention also provides an ostomy bag comprising a filter as claimed in claims 1-14.

A filter of the invention may be applied to any suitable area of the ostomy bag. For example, in one embodiment, the filter is applied to an inner layer of the ostomy bag. In another embodiment, the filter is applied to an outer layer of the ostomy bag.

### Detailed Description

For the purposes of clarity and a concise description, features are described herein as part of the same or separate embodiments; however it will be appreciated that the scope of the invention may include embodiments having combinations of all or some of the features described.

An embodiment of the invention will now be described by way of example with reference to the accompanying figures, in which:-
Figure 1 is a top view of a filter according to the invention.
Figure 2 is a side view of a filter according to the invention.
Figure 3 is a perspective exploded schematic view of a filter according to the invention.
Figure 4 is a perspective view of an ostomy bag including a filter according to the invention.
Figure 5 is an enlarged view of detail A shown in Figure 4.

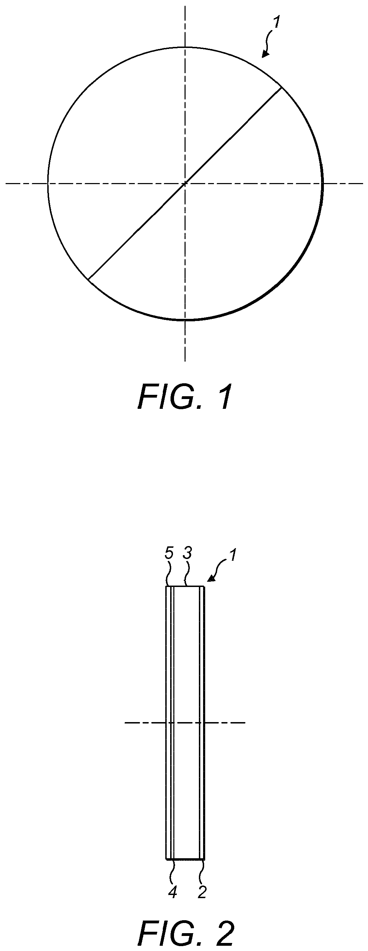

Referring to Figure 1, one embodiment of a filter according to the invention is a disc-shaped filter 1. The disc-shaped filter has a diameter of 24.4 mm and a thickness of 2.7 mm.

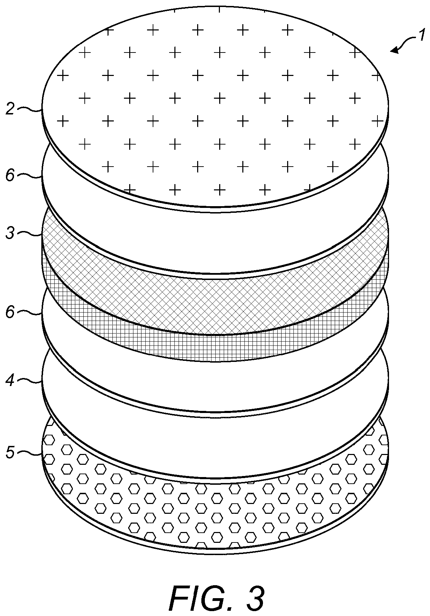

As shown in Figures 2 and 3, the filter 1 comprises a backing layer 2, a layer of odour-adsorbing material 3, a hydrophobic membrane layer 4, and a layer of adhesive webbing 5.

As shown in Figure 3, an adhesive layer 6 is provided between backing layer 2 and the layer of odour-adsorbing material 3. Another adhesive layer 6 is provided between the layer of odour-adsorbing material 3 and the hydrophobic membrane layer 4.

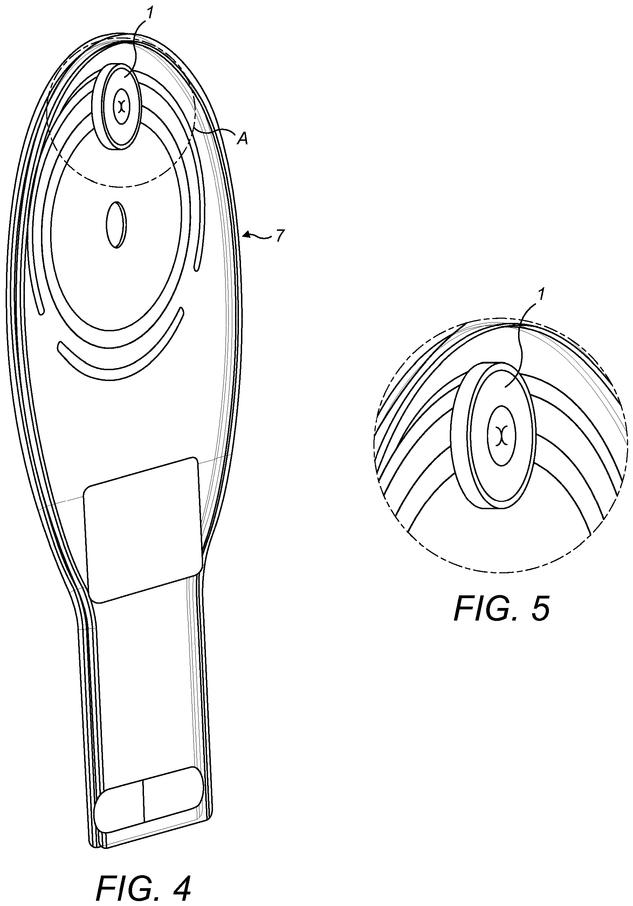

As shown in Figures 4 and 5, the filter 1 may be affixed to an ostomy bag 7, by adhering the layer of adhesive webbing 5 of filter 1 to a section of the ostomy bag 7.

The following Examples illustrate the invention.

### Example: Leak Testing of Ostomy Bags

A disc-shaped filter according to the invention having a 24.5mm diameter was produced and affixed to a standard ostomy bag (bag 1).

Two comparative products (bags 2A and 2B) were produced by affixing a disc-shaped filter having a 24.5mm diameter with no adhesive webbing layer to standard ostomy bags using standard welding techniques. Ostomy bags 2A and 2B are of a similar construction with the only difference between them being the diameter of the exit vents.

The three types of ostomy bags fitted with filters were filled with 250 ml of a water/surfactant/food colouring solution and subjected to a compressive load of 10.5 kg for two minutes. Observation was undertaken to check for leakage through the membrane, at the weld area, and/or around the weld area. The test was repeated using 5-6 ostomy bags of each type.

**Leak Test Results for Ostomy bag 1 (including a filter according to the invention)**

| | Ostomy Bag 1 |
|---|---|
| | |
| 1 | No Leak |
| 2 | No Leak |
| 3 | No Leak |
| 4 | No Leak |
| 5 | No Leak |
| 6 | No Leak |

**Leak Test Results for Ostomy bag 2 (including a standard filter not having the adhesive webbing layer)**

| | Ostomy Bag 2 | | |
|---|---|---|---|
| | A | B | Comments |
| 1 | No Leak | No Leak | No leak - fluid not passing through membrane or around weld periphery. |
| 2 | No Leak | Leak at weld | |
| 3 | Slight Leak | No Leak | Leak - fluid passing through membrane or around weld periphery |
| 4 | Slight Leak | - | |
| 5 | Slight Leak | Leak | |

The above described embodiments have been given by way of example only. It will of course be understood that many variations may be made to the above-described embodiments without departing from the scope of the present i invention as defined by the following claims.

## Claims

1. A filter (1) for an ostomy bag (7) comprising a backing layer (2), a layer of odour-adsorbing material (3), a hydrophobic membrane layer (4), and a layer of adhesive webbing (5), wherein the layer of adhesive webbing comprise distinct fine lines of adhesive, whereby the purpose of the adhesive is to bond the filter (1) to a layer of the ostomy bag (7) with which it is to be used.

2. A filter (1) according to claim 1, wherein the backing layer (2) comprises a fabric layer, a crushed foam layer, a film layer, a non-woven fabric layer, thermally bonded non-woven fabric layer, or any combination thereof.

3. A filter (1) according to claim 1 or claim 2, wherein the backing layer (2) has a thickness of from 5 µm to 200 µm.

4. A filter (1) according to any one of claims 1 to 3, wherein the backing layer (2) is permeable, semi-permeable, or impermeable.

5. A filter (1) according to any one of claims 1 to 4, wherein the layer of odour-adsorbing material (3) comprises activated carbon.

6. A filter (1) according to any one of claims 1 to 5, wherein the layer of odour-adsorbing material (3) comprises a material selected from an activated carbon-impregnated non-woven fabric, an activated carbon-impregnated woven fabric, an activated carbon-impregnated cloth, an activated carbon-impregnated felt, an activated carbon-impregnated knitted fabric, an activated carbon cloth, an activated carbon felt, an activated carbon fabric, an activated carbon-impregnated foam, a polyurethane foam sprayed with chemically activated carbon, a treated activated carbon, a blend of treated activated carbon and activated carbon, and any combination thereof.

7. A filter (1) according to any one of claims 1 to 6, wherein the layer of odour-adsorbing material (3) has a thickness of from about 0.1 to about 5 mm.

8. A filter (1) according to any one of claims 1 to 7, wherein the hydrophobic membrane layer (4) comprises a material that is hydrophobic and oleophobic.

9. A filter (1) according to any one of claims 1 to 8, wherein the hydrophobic membrane layer (4) comprises a material selected from polytetrafluoroethylene (PTFE), expanded PTFE (ePTFE), polyvinylidene difluoride (PVdF), polysulfone (PSu), polyethersulfone (PES), acrylic, nylon, polyoxymethylene (POM) (also known as acetal), a polyolefin, cellulose, and any combination thereof.

10. A filter (1) according to any one of claims 1 to 9, wherein the hydrophobic membrane layer (4) has a thickness of from about 25 µm to about 200 µm and/or comprises a porous material having an average pore size of at least about 0.05 µm up to about 5.0 µm and/or further comprises a support material.

11. A filter (1) according to any one of claims 1 to 10, wherein the adhesive webbing layer (5) comprises a hot melt adhesive and/or a vinyl acetate adhesive.

12. A filter (1) according to any one of claims 1 to 11, further comprising a layer of adhesive (6) provided between the backing layer (2) and the layer of odour-adsorbing material (3), and/or a layer of adhesive (6) provided between the layer of odour-adsorbing material (3) and the hydrophobic membrane layer (4); optionally wherein the or each layer of adhesive (6) comprises a polyamide adhesive.

13. A filter (1) according to any one of claims 1 to 12, wherein the filter (1) is disc-shaped; optionally wherein the disc-shaped filter preferably has a diameter of from about 15 mm to about 40 mm.

14. A filter (1) according to any one of claims 1 to 13, wherein the filter (1) has an overall thickness of from about 2 mm to about 4 mm.

15. An ostomy bag (7) comprising a filter (1) according to any one of claims 1 to 14; optionally wherein the filter (1) is affixed to an inner layer or an outer layer of the ostomy bag (7) and/or wherein the ostomy bag (7) is flushable.

## Patentansprüche

1. Filter (1) für einen künstlichen Darmausgangs-Beutel (7) welcher umfasst, eine Trägerschicht (2), eine Schicht aus einem Geruch-absorbierenden Material (3), eine hydrophobe Membran-Schicht (4), und eine Schicht aus einem klebenden Gewebe (5), worin die Schicht des klebenden Gewebes getrennte feine Klebstofflinien umfasst, wobei der Zweck des Klebstoffes darin besteht den Filter (1) an eine Schicht des künstlichen Darmausgangs-Beutel (7) zu binden, mit dem er verwendet wird

2. Filter (1) nach Anspruch 1, worin die Trägerschicht (2) eine Gewebelage, eine Schicht aus zerkleinertem Schaum, eine Folien-Schicht, eine Vliesstoff-Schicht, eine thermisch gebondete Vliesstoff-Schicht, oder jede Kombination davon umfasst.

3. Filter (1) nach Anspruch 1 oder Anspruch 2, worin die Trägerschicht (2) eine Dicke von 5 µm bis 200 µm aufweist.

4. Filter (1) nach einem der Ansprüche 1 bis 3, worin die Trägerschicht (2) durchlässig, halb-durchlässig oder undurchlässig ist.

5. Filter (1) nach einem der Ansprüche 1 bis 4, worin die Schicht des Geruch-absorbierenden Materials (3) Aktivkohle umfasst.

6. Filter (1) nach einem der Ansprüche 1 bis 5, worin die Schicht des Geruch-absorbierenden Materials (3) ein Material umfasst, ausgewählt unter einem mit Aktivkohle imprägniertem Vliesstoff, einem mit Aktivkohle imprägniertem Gewebe, einem mit Aktivkohle imprägniertem Tuch, einem mit Aktivkohle imprägniertem Filz, einer mit Aktivkohle imprägnierten Maschenware, einem Aktivkohle-Tuch, einem Aktivkohle-Filz, einem Aktivkohle-Gewebe, einem mit Aktivkohle imprägniertem Schaum, einem mit chemisch aktivierter Aktivkohle gesprühtem Polyurethan-Schaum, einer behandelten Aktivkohle, einer Mischung von behandelter Aktivkohle und Aktivkohle, und jeder Kombination davon.

7. Filter (1) nach einem der Ansprüche 1 bis 6, worin die Schicht des Geruch-absorbierenden Materials (3) eine Dicke von etwa 0,1 bis etwa 5 mm aufweist.

8. Filter (1) nach einem der Ansprüche 1 bis 7, worin die hydrophobe Membran Schicht (4) ein Material umfasst, das hydrophob und oleophob ist.

9. Filter (1) nach einem der Ansprüche 1 bis 8, worin die hydrophobe Membran-Schicht (4) ein Material umfasst, ausgewählt unter Polytetrafluoroethylen (PTFE), expandiertem PTFE (ePTFE), Polyvinylidendifluorid (PVdF), Polysulfon (PSu), Polyethersulfon (PES), Acryl, Nylon, Polyoxymethylen (POM) (auch als Acetal bekannt), einem Polyolefin, Cellulose und jeder Kombination davon.

10. Filter (1) nach einem der Ansprüche 1 bis 9, worin die hydrophobe Membran-Schicht (4) eine Dicke von etwa 25 µm bis etwa 200 µm aufweist und/oder ein poröses Material mit einer durchschnittlichen Porengröße von mindestens etwa 0,05 µm bis zu etwa 5,0 µm und/oder weiter ein Trägermaterial umfasst.

11. Filter (1) nach einem der Ansprüche 1 bis 10. worin die Schicht aus klebendem Gewebe (5) einen Heißschmelz-Klebstoff und/oder einen Vinylacetat-Klebstoff umfasst.

12. Filter (1) nach einem der Ansprüche 1 bis 11, welcher weiter eine zwischen der Trägerschicht (2) und der Schicht des Geruch-absorbierenden Material (3) vorgesehene Klebstoffschicht (6) umfasst, und/oder eine zwischen der Schicht des Geruch-absorbierenden Materials (3) und der hydrophoben Membran-Schicht (4) vorgesehene Klebstoffschicht (6); wahlweise worin die oder jede Klebstoffschicht (6) einen Polyamid-Klebstoff umfasst.

13. Filter (1) nach einem der Ansprüche 1 bis 12, worin der Filter (1) scheibenförmig ist; wahlweise worin der scheibenförmige Filter vorzugsweise einen Durchmesser von etwa 15 mm bis etwa 40 mm aufweist.

14. Filter (1) nach einem der Ansprüche 1 bis 13, worin der Filter (1) eine Gesamtdicke von etwa 2 mm bis etwa 4 mm aufweist.

15. Beutel (7) für einen künstlichen Darmausgang, welcher einen Filter (1) nach einem der Ansprüche 1 bis 14 umfasst; wahlweise worin der Filter (1) an einer inneren Schicht oder einen äußeren Schicht des Beutels (7) für einen künstlichen Darmausgang befestigt ist und/oder worin der Beutel (7) für einen künstlichen Darmausgang spülbar ist.

## Revendications

1. Filtre (1) pour un sac de stomie (7) comprenant une couche de support (2), une couche de matériau absorbant les odeurs (3), une couche de membrane hydrophobe (4) et une couche de toile adhésive (5), la couche de toile adhésive comprenant de fines lignes distinctes d'adhésif, ce par quoi le but de l'adhésif est de lier le filtre (1) à une couche du sac de stomie (7) avec lequel il doit être utilisé.

2. Filtre (1) selon la revendication 1, dans lequel la couche de support (2) comprend une couche de tissu, une couche de mousse écrasée, une couche de film, une couche de tissu non tissé, une couche de tissu non tissé thermocollée ou toute combinaison de celles-ci.

3. Filtre (1) selon la revendication 1 ou la revendication 2, dans lequel la couche de support (2) a une épaisseur de 5 µm à 200 µm.

4. Filtre (1) selon l'une quelconque des revendications 1 à 3, dans lequel la couche de support (2) est perméable, semi-perméable ou imperméable.

5. Filtre (1) selon l'une quelconque des revendications 1 à 4, dans lequel la couche de matériau absorbant les odeurs (3) comprend du charbon actif.

6. Filtre (1) selon l'une quelconque des revendications 1 à 5, dans lequel la couche de matériau absorbant les odeurs (3) comprend un matériau choisi parmi un tissu non tissé imprégné de charbon actif, un tissu tissé imprégné de charbon actif, une étoffe imprégnée de charbon actif, un feutre imprégné de charbon actif, un tissu tricoté imprégné de charbon actif, une étoffe de charbon actif, un feutre de charbon actif, un tissu de charbon actif, une mousse imprégnée de charbon actif, une mousse de polyuréthane pulvérisée avec du charbon activé chimiquement, un charbon actif traité, un mélange de charbon actif traité et de charbon actif et toute combinaison de ceux-ci.

7. Filtre (1) selon l'une quelconque des revendications 1 à 6, dans lequel la couche de matériau absorbant les odeurs (3) a une épaisseur d'environ 0,1 à environ 5 mm.

8. Filtre (1) selon l'une quelconque des revendications 1 à 7, dans lequel la couche de membrane hydrophobe (4) comprend un matériau qui est hydrophobe et oléophobe.

9. Filtre (1) selon l'une quelconque des revendications 1 à 8, dans lequel la couche de membrane hydrophobe (4) comprend un matériau choisi parmi le polytétrafluoroéthylène (PTFE), le PTFE expansé (ePTFE), le poly(difluorure de vinylidène) (PVdF), la polysulfone (PSu), la polyéthersulfone (PES), l'acrylique, le nylon, le polyoxyméthylène (POM) (également connu comme acétal), une polyoléfine, la cellulose et toute combinaison de ceux-ci.

10. Filtre (1) selon l'une quelconque des revendications 1 à 9, dans lequel la couche de membrane hydrophobe (4) a une épaisseur d'environ 25 µm à environ 200 µm et / ou comprend un matériau poreux ayant une taille moyenne de pore d'au moins environ 0,05 µm jusqu'à environ 5,0 µm et / ou comprend en outre un matériau de support.

11. Filtre (1) selon l'une quelconque des revendications 1 à 10, dans lequel la couche de toile adhésive (5) comprend un adhésif thermofusible et / ou un adhésif d'acétate de vinyle.

12. Filtre (1) selon l'une quelconque des revendications 1 à 11, comprenant en outre une couche d'adhésif (6) disposée entre la couche de support (2) et la couche de matériau adsorbant les odeurs (3), et / ou une couche d'adhésif (6) disposée entre la couche de matériau absorbant les odeurs (3) et la couche de membrane hydrophobe (4) ; facultativement dans lequel la ou chaque couche d'adhésif (6) comprend un adhésif polyamide.

13. Filtre (1) selon l'une quelconque des revendications 1 à 12, dans lequel le filtre (1) est en forme de disque ; facultativement dans lequel le filtre en forme de disque a de préférence un diamètre d'environ 15 mm à environ 40 mm.

14. Filtre (1) selon l'une quelconque des revendications 1 à 13, dans lequel le filtre (1) a une épaisseur globale d'environ 2 mm à environ 4 mm.

15. Sac de stomie (7) comprenant un filtre (1) selon l'une quelconque des revendications 1 à 14 ; facultativement dans lequel le filtre (1) est fixé à une couche intérieure ou une couche extérieure du sac de stomie (7) et / ou dans lequel le sac de stomie (7) est jetable dans les toilettes.
